# EUROPEAN PATENT APPLICATION

(11) **EP 4 600 346 A1**
(43) Date of publication of application: **13.08.2025**
(21) Application number: 23875244.8
(22) Date of filing: 05.10.2023
(51) Int. Cl.: C12N 5/071, A61K 35/55, A61P 1/02

(54) **MEDIUM AND METHOD FOR PREPARATION OF SALIVARY GLAND ORGANOID**

(30) Priority: 05.10.2022 KR 20220127416
(71) Applicant: Organoidsciences Ltd., Seongnam-si, Gyeonggi-do 13493 (KR)
(72) Inventor: LEE, Jaeseon, Seongnam-si, Gyeonggi-do 13488 (KR); SEO, Jaehwi, Seongnam-si, Gyeonggi-do 13488 (KR); CHOI, Jinkyoung, Seongnam-si, Gyeonggi-do 13488 (KR); KIM, Yeeun, Seongnam-si, Gyeonggi-do 13488 (KR)
(74) Representative: Rückerl, Florian
(86) International application number: PCT/KR2023/015359
(87) International publication number: WO 2024/076182

(57) **Abstract**

The present invention relates to a medium composition for preparing a salivary gland organoid. Also, the present invention relates to: a method for preparing a salivary gland organoid, comprising a step of culturing salivary gland-derived cells in the medium composition; and a salivary gland organoid prepared by the preparation method. A medium for preparing a salivary gland organoid, according to the present invention, can promote the proliferation of a salivary gland organoid, maintain the stemness of the salivary gland organoid, and increase engraftment capability. Therefore, the medium can be used to prepare a large amount of salivary gland organoids with excellent quality, and salivary gland organoids prepared using the medium can be used as a preventive or therapeutic agent for salivary gland-related diseases, an alternative to animal experimental models for salivary gland-related diseases, or the like.

## Description

### [Technical Field]

The present invention relates to a medium composition for preparing salivary gland organoids. The present invention also relates to a method for preparing salivary gland organoids, comprising a step of culturing salivary gland-derived cells using the medium composition, and to salivary gland organoids prepared by the method.

### [Background Art]

Stem cells possess unique characteristics of differentiation ability (multi-potency) and self-renewal, and accordingly, research has been continuously conducted to utilize them in various applications such as treatment of incurable diseases, disease modeling, and transplantation of tissues or organs. In particular, it has been revealed that when stem cells are cultured in an appropriate three-dimensional in vitro environment, cell clusters with structural and tissue characteristics similar to in vivo organs are formed, and such cell clusters are referred to as organoids.

Organoid production technology is theoretically expected to enable the generation of almost all types of organs using stem cells alone, and is anticipated to be utilized in the development of therapeutics for various diseases. Organoids may be more effective in testing the safety and efficacy of new drugs compared to two-dimensional cell tissues, and are considered to be applicable for improving conditions through transplantation into damaged or underdeveloped organs. Accordingly, organoid-related research is becoming increasingly active from the perspective of regenerative medicine, and organoids are anticipated to be widely applicable across various fields.

However, since the technologies for preparing and culturing organoids are still in the early stages of research, various studies are required to establish preparing and culturing methods, such as determining the combination or ratio of medium additives. In this regard, medium compositions for proliferative liver organoid differentiation and methods for producing liver organoids using the same (Korean Patent Publication No. 10-2021-0028562), as well as methods for producing brain organoids (Korean Patent No. 10-2228400), have been developed; however, there remains a need to develop medium compositions optimized for each organ and methods for producing organoids using such compositions.

### [Technical Problem]

The inventors conducted extensive research to efficiently prepare high-quality salivary gland organoids. As a result, they identified an optimal combination of medium components that can promote proliferation, maintain stemness, and enhance engraftment capability of salivary gland organoids, and completed the present invention by experimentally demonstrating its effects.

### [Technical Solution]

Each description and embodiment disclosed in the present invention is also applicable to other descriptions and embodiments. That is, all combinations of the various elements disclosed herein fall within the scope of the present invention. In addition, it cannot be considered that the scope of the present invention is limited by the specific descriptions disclosed below.

In addition, it should be understood that terms not specifically defined in this specification have meanings commonly used in the technical field to which the present invention pertains. Also, unless otherwise defined by context, the singular includes the plural, and the plural includes the singular.

One aspect of the present invention provides a medium composition for preparing salivary gland organoids, comprising forskolin (FSK), FGF-10 and heregulin-β1 (HRG-β1).
The medium composition of the present invention can promote the proliferation of salivary gland organoids to form a large number of salivary gland organoids. The formed salivary gland organoids maintain stemness and exhibit enhanced engraftment capability upon in vivo transplantation. Therefore, the medium composition is usefully applicable to the production of salivary gland organoids for various applications such as regenerative therapeutics and drug screening.

As used in the present specification, the term "organoid" refers to a three-dimensional (3D) cell aggregate. The organoid is produced through an artificial culturing process without collection, acquisition, or harvesting from animals or the like, and is defined as a miniaturized and simplified version of an organ. The organoid according to the present invention may be a salivary gland organoid, but is not limited thereto.

As used in the present specification, the term "FSK (forskolin)" refers to a compound also known as "coleonol", which is defined by CAS Number 66575-29-9. The FSK may be contained in the medium composition of the present invention at a concentration of 0.1 to 10 µM, specifically at a concentration of 0.1 to 5 µM, and more specifically at a concentration of 1 µM, but is not limited thereto.

As used in the present specification, the term "FGF-10" refers to fibroblast growth factor 10 protein encoded by the *FGF10* gene. Information regarding its protein sequence and the like may be found in publicly available databases such as NCBI GenBank. The FGF-10 may be contained in the medium composition of the present invention at a concentration of 50 to 150 ng/ml, and more specifically at a concentration of 100 ng/ml, but is not limited thereto.

As used in the present specification, the term "HRG-β1 (heregulin-β1)" refers to a protein also known as "neuregulin 1 (NRG1)", which is encoded by the *NRG1* gene. Information regarding its protein sequence and the like may be found in publicly available databases such as NCBI GenBank. The HRG-β1 may be contained in the medium composition of the present invention at a concentration of 1 to 10 nM, and more specifically at a concentration of 5 nM, but is not limited thereto.

In the present invention, when salivary gland organoids are cultured using a medium containing FSK, FGF-10 and HRG-β1, it was confirmed that the proliferation of the salivary gland organoids was promoted, resulting in a significant increase in the number of salivary gland organoids obtained. Furthermore, it was confirmed that most of the formed organoids maintained stemness and exhibited increased engraftment capability, thereby demonstrating excellent effects in the production of salivary gland organoids.

The sources of FSK, FGF-10, and HRG-β1 are not particularly limited. For example, commercially available materials may be used, or they may be prepared using gene (or protein) recombinant technology according to methods known in the art.

Specifically, the salivary gland organoids according to the present invention may express one or more selected from the group consisting of EpCAM, CD29, E-cadherin and CD49f.

As used in the present specification, the term "EpCAM (epithelial cell adhesion molecule)" refers to a gene used as a salivary gland epithelial cell marker. The term "CD29" refers to a gene also known as "ITGB1 (integrin subunit beta 1)", which is used as a salivary gland epithelial cell marker. The term "E-cadherin" refers to a gene used as a salivary gland epithelial cell marker. The term "CD49f" refers to a gene used as a stem cell marker.

In addition, the medium composition for preparing salivary gland organoids according to the present invention may not comprise one or more selected from the group consisting of FGF-2 and FGF-7. When salivary gland organoids are prepared using a medium composition containing FGF-2 and/or FGF-7, the cultured salivary gland organoids exhibit a reduction in proliferative capacity or fail to exhibit organoid characteristics, such as losing their spheroid morphology.

As used in the present specification, the term "FGF-2" refers to fibroblast growth factor 2 protein encoded by the *FGF2* gene. Information regarding its protein sequence and the like may be found in publicly available databases such as NCBI GenBank. The term "FGF-7" refers to fibroblast growth factor 7 protein encoded by the *FGF7* gene. Information regarding its protein sequence and the like may be found in publicly available databases such as NCBI GenBank.

In addition, the medium composition for preparing salivary gland organoids according to the present invention may not comprise one or more selected from the group consisting of R-spondin and Noggin. When salivary gland organoids are prepared using a medium composition containing R-spondin and/or Noggin, the cultured salivary gland organoids exhibit a reduction in proliferative capacity, or fail to exhibit organoid characteristics, such as losing their spheroid morphology or losing stemness.

As used in the present specification, the term "R-spondin" refers to a protein encoded by the *Rspo1* gene, which promotes WNT/β-catenin signaling. Information regarding its protein sequence and the like may be found in publicly available databases such as NCBI GenBank. The term "Noggin" refers to a protein encoded by the *NOG* gene, which inhibits bone morphogenetic protein (BMP). Information regarding its protein sequence and the like may be found in publicly available databases such as NCBI GenBank.

In the present invention, the salivary gland organoids may be cultured through at least 5 passages, or at least 7 passages.

As used in the present specification, the term "passage" refers to a cycle of replacing the culture vessel or splitting the cell population in order to continuously culture cells or organoids in a healthy state over an extended period of time by successively propagating the cell lineage. One passage refers to a single replacement of the culture vessel or a single splitting of the cell population, whereas seven passages refer to seven replacements of the culture vessel or seven splittings of the cell population. Increasing the number of passages enables the acquisition of a larger number of organoids. However, conventional culture media used in the art have had the problem that as the number of passages increases, the cells constituting the organoids undergo cell death, thereby making long-term culture impossible. In contrast, the medium composition comprising FSK, FGF-10 and HRG-β1 according to the present invention enables long-term culture of salivary gland organoids through at least seven passages. In the present specification, the term "passage" may be used interchangeably with the term "generation".

As used in the present specification, the term "medium" refers to a mixture of nutrients that enables the growth, survival, expansion, or differentiation of cells or organoids in vitro, and includes all appropriate conventional media used in the art. Depending on the type of cell or organoid, the type of medium and culturing conditions may be selected within the technical level of those skilled in the art. The medium may be a basal medium for cell culture containing a carbon source, nitrogen source, and trace elements, and specific examples include, but are not limited to, DMEM (Dulbecco's Modified Eagle's Medium), MEM (Minimal Essential Medium), BME (Basal Medium Eagle), RPMI1640, F-10, F-12, α-MEM (α-Minimal Essential Medium), GMEM (Glasgow's Minimal Essential Medium), IMDM (Iscove's Modified Dulbecco's Medium), DMEM/F12 (Dulbecco's Modified Eagle Medium/Nutrient Mixture F-12), or Advanced DMEM/F12.

In the conventional methods for preparing organoids, at least two types of culture media having different components have been used. For example, a growth medium has been used in the initial stage to form organoids and promote their growth in size, followed by the use of an expansion medium, after removing the growth medium, to increase the number of organoids. However, the conventional methods using two or more types of culture media with different components have disadvantages in that they are costly and make the preparation process complicated.

In addition, due to their inherent characteristics, organoids exhibit significantly different preparation outcomes depending on the culture conditions. Examples of culture conditions include culture duration, culture temperature, culture medium, and culture matrix, all of which are important factors in determining the properties of the organoids. Among them, the medium conditions play the most critical role in enabling organoids to grow with characteristics similar to the desired tissue or organ by regulating various signaling pathways in stem cells.

In the present invention, it was confirmed that the optimal medium conditions for obtaining a sufficient number of salivary gland organoids that are highly similar to actual tissue in morphology and function, and in particular have enhanced proliferative capacity and stemness enabling their use in regenerative therapeutics or drug screening, comprise FSK, FGF-10 and HRG-β1. Furthermore, it was confirmed that when using the medium described above, salivary gland organoid formation, growth, and expansion can be successfully induced using only a single type of culture medium, without the need for two or more types of culture media.

In the present specification, the term "for preparation" may be used interchangeably with the term "for culturing." Accordingly, the medium composition for preparing salivary gland organoids may be a medium composition for culturing salivary gland organoids, a medium composition for forming salivary gland organoids, or a medium composition for expanding salivary gland organoids. Here, the term "for forming" refers to forming salivary gland organoids from salivary gland-derived cells and enabling the organoids to exhibit organ or tissue characteristics so as to perform the inherent functions of the salivary gland. In addition, the term "for expanding" refers to increasing the number of formed salivary gland organoids to obtain a sufficient quantity for the intended purpose, meaning that the organoids can be passaged for at least two generations.

In the present invention, the medium composition may further comprise an antibiotic, and the antibiotic includes any conventional antibiotic appropriately used in the art for culturing cells or organoids. Specific examples may be one or more selected from the group consisting of gentamicin, amphotericin, penicillin and streptomycin, but are not limited thereto.

Another aspect of the present invention provides a method for preparing salivary gland organoids, comprising a step of culturing salivary gland-derived cells isolated from a subject using the medium composition described above.

In the method for preparing salivary gland organoids according to the present invention, unless otherwise specified, each term has the same meaning as described above in relation to the medium composition for preparing salivary gland organoids.

As used in the present specification, the term "subject" includes a human or any non-human animal without limitation. The non-human animal may be a vertebrate, for example, a primate, dog, cow, horse, pig, or rodent, such as a mouse, rat, hamster, or guinea pig. In the present specification, the term "subject" may be used interchangeably with the term "individual" or "patient".

The isolated salivary gland-derived cells may be obtained through a tissue dissection process and an enzymatic digestion process. Specifically, the dissection includes both physical dissection and mechanical dissection, and may be performed using general tissue dissection methods known in the art. The enzymatic digestion may be performed under general enzymatic digestion conditions known in the art, and may be carried out using one or more enzymes selected from the group consisting of liberase, dispase II, deoxyribonuclease (DNase) I and collagenase II.

In the present invention, the culturing may be performed through at least seven passages, and one passage may be carried out over a period of 1 to 10 days.

In addition, culturing conditions such as culturing period, culturing temperature and culturing substrate may be selected from conditions conventionally used in the art for culturing organoids, and those skilled in the art will be able to employ methods suitable for the purpose of the present invention.

Another aspect of the present invention provides salivary gland organoids prepared by the method for preparing salivary gland organoids as described above.

In the salivary gland organoids according to the present invention, unless otherwise specified, each term has the same meaning as described above in relation to the medium composition for preparing salivary gland organoids.

The salivary gland organoids according to the present invention maintain stemness and have a histological structure and function highly similar to those of the human salivary gland. When administered in vivo, they can engraft into damaged areas of the salivary gland and promote regeneration thereof. Accordingly, the salivary gland organoids can be used as an agent for the prevention or treatment of salivary gland-related diseases such as sialadenitis, xerostomia, salivary gland tumors, salivary gland damage caused by radiation or radioactive isotopes, and age-related decline in salivary gland function. In addition, they can be used as an alternative to animal models for salivary gland-related diseases.

As used in the present specification, the term "treatment" means any act of improving or curing salivary gland dysfunction-related symptoms through transplantation or administration of the salivary gland organoids according to the present invention. The term "prevention" means any act of suppressing or delaying the onset of salivary gland dysfunction-related symptoms through transplantation or administration of the salivary gland organoids according to the present invention.

Another aspect of the present invention provides a pharmaceutical composition for the prevention or treatment of salivary gland diseases, comprising the salivary gland organoids described above.

In the pharmaceutical composition according to the present invention, unless otherwise specified, each term has the same meaning as described above.

The pharmaceutical composition according to the present invention may be a cell therapy composition.

As used in the present specification, the term "cell therapy agent" refers to a type of regenerative therapeutic agent, which is a pharmaceutical agent prepared from living autologous, allogeneic or xenogeneic cells for purposes such as prevention, treatment or diagnosis. The cell therapy agent may be used for the regeneration of damaged or defective cells or tissues and may be used to restore the function and morphology of damaged or defective cells or tissues.

The pharmaceutical composition according to the present invention may comprise an effective amount of salivary gland organoids and may be administered to a subject in need of prevention or treatment of salivary gland diseases.

As used in the present specification, the term "salivary gland disease" refers to a condition in which the salivary gland tissue is damaged or inflamed, thereby impairing its ability to perform normal functions.

The salivary gland diseases that can be prevented or treated by the pharmaceutical composition of the present invention may be one or more selected from the group consisting of sialadenitis, xerostomia, salivary gland tumors, salivary gland damage caused by radiation or radioactive isotopes, and age-related decline in salivary gland function, but are not limited thereto. In addition, the sialadenitis may include one or more selected from the group consisting of tuberculous sialadenitis, infectious acute sialadenitis, infectious chronic sialadenitis and sialadenitis caused by radiation or radioactive isotopes, but is not limited thereto. In this context, an example of the radioactive isotope may be radioactive iodine, but is not limited thereto.

The effective amount may be a "therapeutically effective amount" or a "prophylactically effective amount". As used in the present specification, the term "therapeutically effective amount" refers to any amount of a drug or therapeutic agent that, when used alone or in combination with other therapeutic agents, results in a reduction in the severity of disease symptoms, an increase in the frequency or duration of symptom-free periods, or prevention of damage or disability caused by the disease. The term "prophylactically effective amount" refers to any amount that suppresses the occurrence or recurrence of salivary gland diseases in a subject. The level of the effective amount may be determined based on factors such as the severity of the condition, age, sex, activity of the drug, sensitivity to the drug, timing of administration, route of administration, rate of excretion, duration of treatment, concomitant drugs, and other factors well known in the medical field, in the subject.

As used in the present specification, the term "administration" refers to the physical introduction of the composition into a subject using any of a variety of methods and delivery systems well known to those skilled in the relevant art. Routes of administration for the pharmaceutical composition of the present invention include, but are not limited to, submucosal administration, intramuscular, subcutaneous, intraperitoneal, spinal, or other non-oral routes such as administration by injection or infusion. In addition, non-surgical administration using a device such as a catheter is possible, as well as surgical administration methods such as injection or implantation following incision of the diseased area. The frequency of administration of the composition of the present invention may include, for example, a single administration, multiple administrations, or one or more administrations over an extended period.

The administration of pharmaceutical composition of the present invention may vary depending on the age, sex, and body weight of the subject. Specifically, it may be administered once to several times per day, or at intervals of several days to several months, depending on the symptoms of the subject. In addition, the dose may be adjusted such that the salivary gland organoids comprised in the pharmaceutical composition of the present invention are administered at 5×10⁵/cm² to 5×10⁶ cells/cm², but is not limited thereto, and may be increased or decreased depending on factors such as the route of administration, severity of the disease, sex, body weight, and age.

The pharmaceutical composition of the present invention may further comprise a suitable carrier, excipient, or diluent conventionally used in the preparation of pharmaceutical compositions. Examples of carriers, excipients, and diluents that may be comprised in the composition include, but are not limited to, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylparaben, propylparaben, talc, magnesium stearate, and mineral oil.

As used in the present specification, the term "subject" includes a human and a non-human animal, and the non-human animal includes, for example, a vertebrate such as a primate, dog, cow, horse, pig, or rodent such as a mouse, rat, or guinea pig. In the present specification, the term "subject" may be used interchangeably with the terms "individual" and "patient".

The pharmaceutical composition of the present invention may be administered in combination with other therapeutic agents. In such cases, the pharmaceutical composition of the present invention and the other therapeutic agents may be administered simultaneously, sequentially, or separately. The other therapeutic agents may be drugs such as compounds or proteins having preventive, therapeutic, or ameliorative effects on salivary gland diseases, but are not limited thereto.

In addition, the pharmaceutical composition of the present invention may be formulated to allow simultaneous, sequential, or separate administration with other therapeutic agents. For example, the salivary gland organoids and the other therapeutic agents may be administered simultaneously as a single formulation, or may be administered simultaneously, sequentially, or separately as separate formulations. For simultaneous, sequential, or separate administration, the salivary gland organoids and the other therapeutic agents comprised in the pharmaceutical composition of the present invention may be formulated in separate containers or in a single container. Furthermore, the salivary gland organoids and the other therapeutic agents comprised in the pharmaceutical composition of the present invention may have the same or different pharmaceutical effective amounts, administration times, administration intervals, administration routes, or treatment durations.

Another aspect of the present invention provides a method for the prevention or treatment of salivary gland diseases, comprising a step of administering the salivary gland organoids to a subject in need of prevention or treatment of salivary gland diseases.

In the method for the prevention or treatment of salivary gland diseases according to the present invention, unless otherwise specified, each term has the same meaning as described above in relation to the medium composition for preparing salivary gland organoids and the pharmaceutical composition for the prevention or treatment of salivary gland diseases.

In the method for the prevention or treatment of salivary gland diseases according to the present invention, the salivary gland organoids may be administered to the subject simultaneously, sequentially, or separately with other therapeutic agents.

The term "simultaneous" administration as used herein refers to administration of the salivary gland organoids and other therapeutic agents at the same time as a single formulation. It also refers to administration of the salivary gland organoids and other therapeutic agents at the same time as separate formulations, in which case the routes of administration of the salivary gland organoids and the other therapeutic agents may differ.

The term "sequential" administration as used herein refers to administration of the salivary gland organoids and other therapeutic agents in a relatively continuous manner, allowing only the minimum time required between administrations.

The term "separate" administration as used herein refers to administration of the salivary gland organoids and other therapeutic agents with a predetermined time interval between administrations. The methods of administration of the salivary gland organoids and other therapeutic agents may be appropriately selected by a physician or expert in the art in consideration of therapeutic efficacy, side effects, and other factors in the subject.

The medium for preparing salivary gland organoids according to the present invention can promote the proliferation of salivary gland organoids, maintain the stemness of the salivary gland organoids, and enhance their engraftment capability. Accordingly, the medium can be used to produce a large quantity of high-quality salivary gland organoids, and the salivary gland organoids prepared using the medium can be utilized as preventive or therapeutic agents for salivary gland diseases, or as alternatives to animal models for salivary gland diseases.

### [Brief Description of Figures]

FIG. 1 is an image showing salivary gland organoids cultured using the medium of the present invention. "P" represents passage, and "D" represents culture day; for example, P0D1 indicates the first day of culture in passage 0. Salivary gland organoids were cultured using salivary gland tissues obtained from two different patients.
FIG. 2a is a graph showing the number of salivary gland organoids cultured using the medium of the present invention according to passage.
FIG. 2b is a graph showing the size of salivary gland organoids cultured using the medium of the present invention.
FIG. 3 is an image showing the results of chromosome karyotype analysis of salivary gland organoids cultured using the medium of the present invention.
FIG. 4a is a graph showing the expression level of EpCAM in salivary gland organoids cultured with the medium of the present invention.
FIG. 4b is a graph showing the expression level of CD49f in salivary gland organoids cultured with the medium of the present invention.
FIG. 4c is a graph showing the expression level of CD29 in salivary gland organoids cultured with the medium of the present invention.
FIG. 4d is a graph showing the expression level of E-cadherin in salivary gland organoids cultured with the medium of the present invention.
FIG. 5a is a schematic diagram illustrating the preparation of an animal model of radiation-induced sialadenitis and the in vivo transplantation process of salivary gland organoids cultured with the medium of the present invention.
FIG. 5b is an image confirming the engraftment of salivary gland organoids cultured with the medium of the present invention, showing the results of hematoxylin and eosin staining 4 weeks after transplantation.
FIG. 5c is an image confirming the engraftment of salivary gland organoids cultured with the medium of the present invention, showing the results of immunofluorescence staining for E-cadherin, nuclei, and Hoechst 4 weeks after transplantation (green: human E-cadherin; red: human nuclei; blue: Hoechst).
FIG. 5d is an image confirming the engraftment of salivary gland organoids cultured with the medium of the present invention, showing the results of immunofluorescence staining for human cytokeratin 19 (CK19) and Hoechst 4 weeks after transplantation (green: human CK19; blue: Hoechst).
FIG. 6a is an image showing salivary gland organoids cultured with the medium of the present invention (FSK(+)) or with a medium excluding FSK from the medium composition of the present invention (FSK(-)).
FIG. 6b is a graph showing the proliferative capacity of salivary gland organoids cultured with the medium of the present invention (FSK(+)) or with a medium excluding FSK from the medium composition of the present invention (FSK(-)).
FIG. 7a is an image showing salivary gland organoids cultured with the medium of the present invention (FGF-2(-)) or with a medium in which FGF-2 is contained in the medium composition of the present invention (FGF-2(+)).
FIG. 7b is a graph showing the proliferative capacity of salivary gland organoids cultured with the medium of the present invention (FGF-2(-)) or with a medium in which FGF-2 is contained in the medium composition of the present invention (FGF-2(+)).
FIG. 8a is an image showing salivary gland organoids cultured with the medium of the present invention (FGF-7(-)) or with a medium in which FGF-7 is contained in the medium composition of the present invention (FGF-7(+)).
FIG. 8b is a graph showing the proliferative capacity of salivary gland organoids cultured with the medium of the present invention (FGF-7(-)) or with a medium in which FGF-7 is contained in the medium composition of the present invention (FGF-7(+)).
FIG. 9a is an image showing salivary gland organoids cultured with the medium of the present invention (FGF-10(+)) or with a medium excluding FGF-10 from the medium composition of the present invention (FGF-10(-)).
FIG. 9b is a graph showing the proliferative capacity of salivary gland organoids cultured with the medium of the present invention (FGF-10(+)) or with a medium excluding FGF-10 from the medium composition of the present invention (FGF-10(-)).

### [Examples]

Hereinafter, the present invention will be described in more detail with reference to the following Examples. These Examples are provided to illustrate the present invention in greater detail and are not intended to limit the scope of the present invention.

### Example 1. Establishment of Medium Composition for Preparation of Salivary Gland Organoids

### Example 1-1. Method for Preparing Salivary Gland Organoids

To develop a medium composition for efficiently preparing salivary gland organoids, the optimal combination of components constituting the medium composition was established as shown in Table 1 below. In this experiment, Advanced DMEM/F12 was used as the basal medium, and gentamicin was used as an antibiotic at a concentration of 10 µg/mL.

**[Table 1]**

| Medium Composition | Concentration |
|---|---|
| GlutaMAX^{™} Supplement | 1x |
| B27^{™} Supplement (50x), Serum-free | 1x |
| HEPES, 1M | 10 mM |
| Y-27632 | 10 µM |
| NAC (N-acetyl-L-cysteine) | 1.25 mM |
| A83-01 | 0.5 µM |
| Nicotinamide | 10 mM |
| FGF-10 | 100 ng/ml |
| HRG-β1 | 5 nM |
| Forskolin | 1 µM |

The culturing of salivary gland organoids using the above medium composition was performed as follows. Salivary gland tissue obtained from patients through surgical resection was washed three times or more with a washing solution containing an antibiotic. The washed salivary gland tissue was finely chopped and treated with liberase at 37°C for 45 minutes to isolate salivary gland stem cells. The isolated stem cells were suspended at 40,000 cells/20 µL in a mixture of collagen matrix and the above culture medium, and 20 µL per well was plated into a 48-well plate and polymerized at 37°C for 30 minutes. The collagen matrix used herein has been reported to be suitable for implantation into the body, and has the advantage of avoiding infection issues and batch-to-batch variability associated with matrigel. Subsequently, 250 µL per well of the above culture medium was added, and three-dimensional culturing was performed in a 37°C, 5% CO₂ incubator. The culture medium was replaced every 2 to 3 days.

Passaging was performed on day 8 at passage 0, and subsequently at 7-day intervals. The collagen matrix containing salivary gland organoids was collected, treated with liberase (0.23 mg/mL) at 37°C for 30 minutes to dissociate the organoids. After centrifugation at 900 × g for 10 minutes, the pellet was treated with 1X TrypLE for 30 minutes to obtain a single-cell suspension. The number and viability of single cells were recorded, and the cells were resuspended at 20,000 cells/20 µL in a mixture of collagen matrix and the above culture medium, followed by three-dimensional culturing in the collagen matrix.

### Example 1-2. Analysis of Cultured Salivary Gland Organoids

Salivary gland organoids prepared according to the method of Example 1-1 were analyzed for cell morphology, proliferative capacity, size, chromosomal abnormalities, expression of salivary gland-specific markers, and in vivo engraftment.

Specifically, the cultured salivary gland organoids were observed and photographed under an optical microscope during the early culture period (days 0-3), the mid-culture period (days 4-5), and just prior to passaging (days 7-8). The number of organoids was determined by dissociating the salivary gland organoids into single cells and counting the number of single cells. The size of the organoids was determined by using the ImageJ program to measure and record the maximum diameter of each of approximately 100 organoids on each culture day. Karyotype analysis was performed on organoids at day 7 of passage 5. The expression of salivary gland epithelial cell markers EpCAM, CD29, and E-cadherin was evaluated by flow cytometry using methods known in the art, and the expression of CD49f was evaluated to confirm stem cell characteristics.

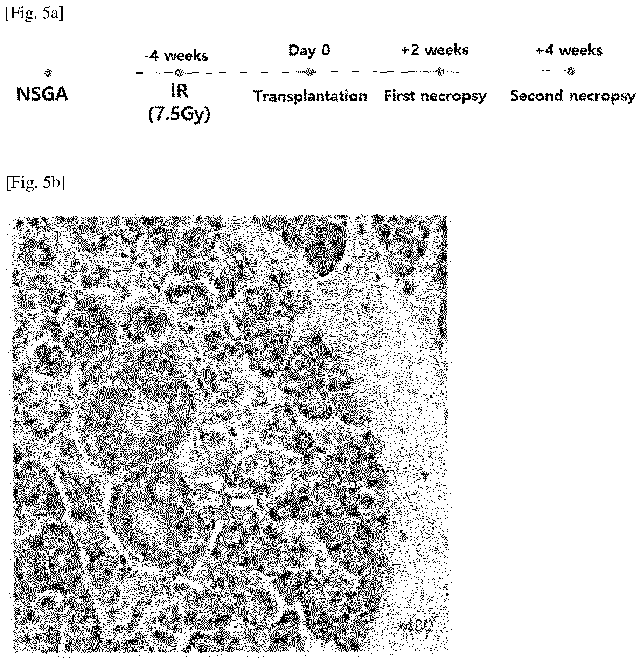

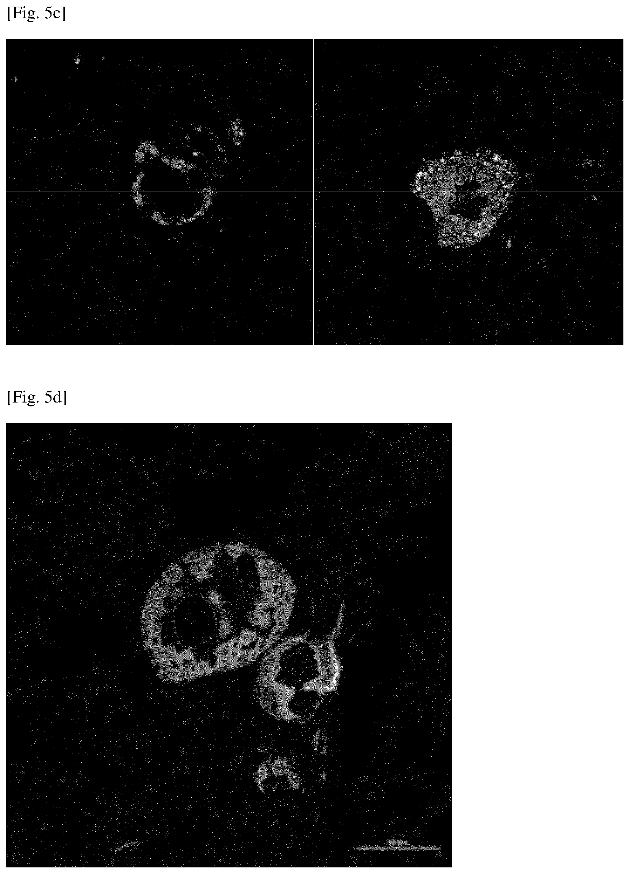

Meanwhile, transplantation of the salivary gland organoids was performed as follows. An immunodeficient NSGA mouse was anesthetized by intraperitoneal injection of a ketamine and Rompun mixture and immobilized. The salivary gland region of the mouse was irradiated at 7.5 Gy to generate a salivary gland injury/sialadenitis animal model induced by radiation. Four weeks after irradiation, approximately 300 human salivary gland organoids prepared at day 7 of passage 1 were directly injected into the salivary glands of the irradiated model in a volume of 20 µL. Four weeks after transplantation, the salivary gland tissue was excised, fixed in 4% paraformaldehyde solution, embedded in paraffin, sectioned into 5 µm thick slices, and subjected to deparaffinization and rehydration. The tissue sections were then stained with hematoxylin and eosin and observed under a microscope at 400× magnification to examine tissue morphology. In addition, immunofluorescence staining was performed on the tissue sections to analyze human nuclei, human E-cadherin, and human cytokeratin 19 (CK19). An overview of the preparation of this sialadenitis animal model is shown in FIG. 5a.

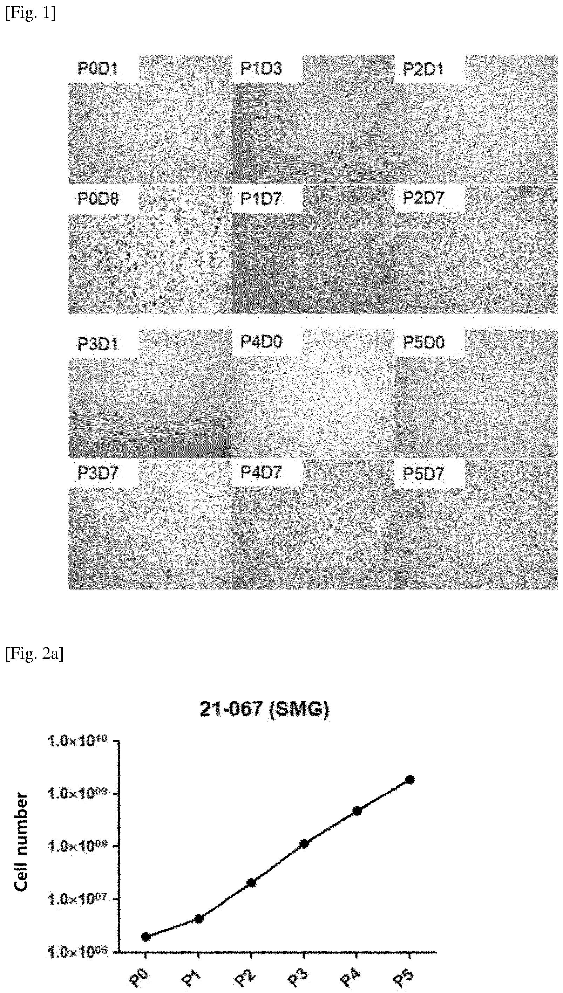

As shown in FIG. 1, the cultured salivary gland organoids proliferated in a spheroid form while maintaining epithelial cell characteristics, exhibiting a three-dimensional structure in which cells were bound to each other through specific cell-cell adhesion proteins (mainly E-cadherin) and were surrounded externally by extracellular matrix up to passage 5.

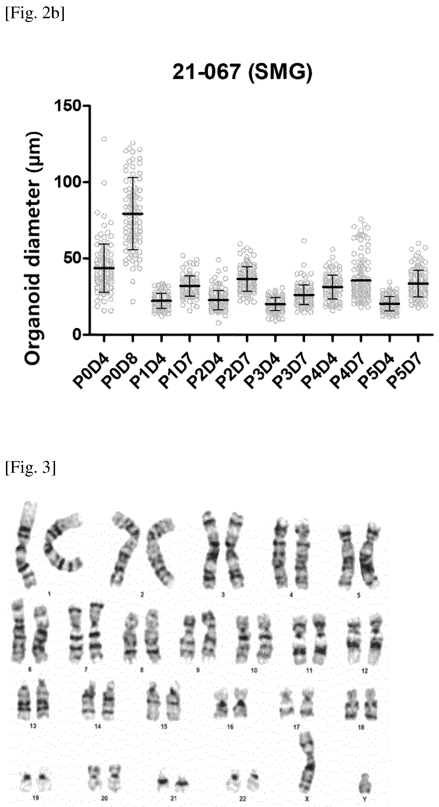

In addition, as shown in FIGS. 2a and 2b, the number of salivary gland organoids continuously increased with each passage, and from passage 1 onward, the organoids maintained a consistent size with a diameter of approximately 32 to 37 µm. These results indicate that salivary gland organoids cultured using the medium composition of the present invention can be produced with uniform quality.

As shown in FIG. 3, the salivary gland organoids were analyzed for chromosomal abnormalities on day 7 of passage 5, and it was found that the consistently observed chromosomes exhibited no numerical or structural abnormalities. This indicates that the salivary gland organoids cultured using the medium composition of the present invention are safe and can be usefully applied for in vivo transplantation.

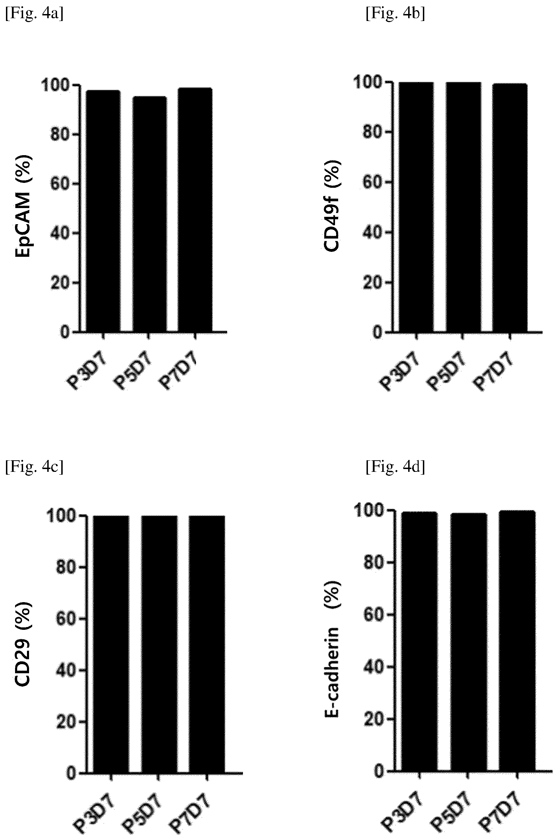

In addition, as shown in FIGS. 4a to 4d, salivary gland organoids maintained a consistent level of expression of the salivary gland organoid markers EpCAM, CD29, and E-cadherin, as well as the stem cell marker CD49f, on day 7 of passage 7, corresponding to a total culture period of 57 days. These results indicate that salivary gland organoids cultured using the medium composition of the present invention still maintain stemness without differentiating into mature cells, even during long-term culture.

Particularly, as shown in FIGS. 5b to 5d, the transplanted human salivary gland organoids were engrafted within the salivary gland tissue of the sialadenitis animal model induced by irradiation, exhibited polarization with strong expression of CK19 in the lumen, and were confirmed to possess morphology and function similar to those of human salivary gland tissue. These results indicate that salivary gland organoids cultured using the medium composition of the present invention can regenerate damaged salivary glands and can be usefully applied for the prevention or treatment of salivary gland-related diseases.

### Example 2. Comparison of Medium Compositions for Salivary Gland Organoids

To evaluate the effects of the medium for preparing salivary gland organoids established in Example 1, medium compositions were prepared by partially modifying the composition disclosed in Table 1.

Specifically, based on the composition of Table 1, (1) a medium without FSK (forskolin), (2) a medium containing FGF-2, (3) a medium containing FGF-7, and (4) a medium without FGF-10 were prepared, and salivary gland organoids were cultured using these media and then analyzed.

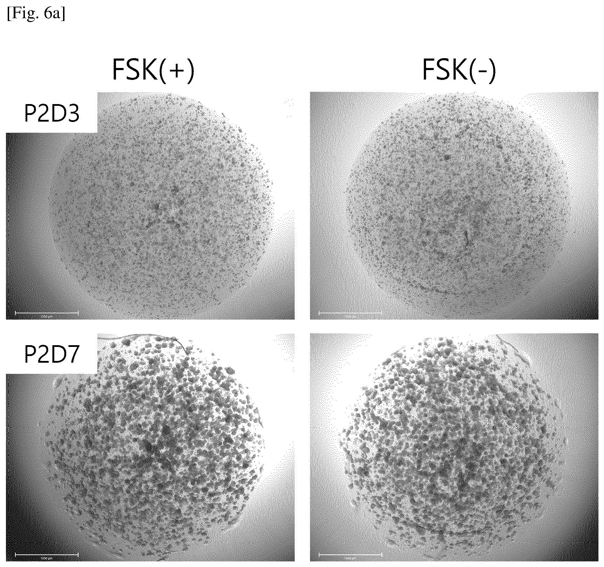

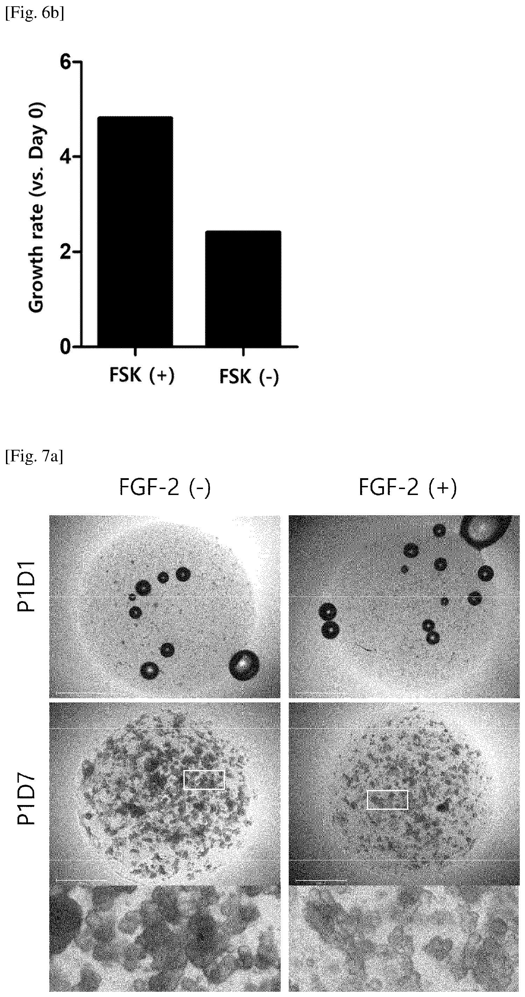

As shown in FIGS. 6a and 6b, salivary gland organoids cultured in the medium without FSK exhibited approximately a two-fold decrease in cell proliferation rate compared to those cultured in the medium containing FSK.

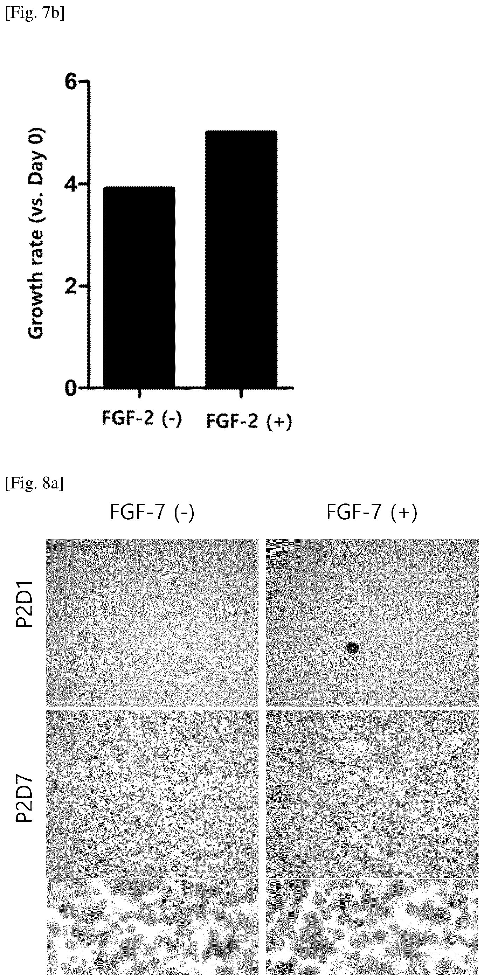

In addition, as shown in FIGS. 7a and 7b, salivary gland organoids cultured in the medium containing FGF-2 exhibited an approximately 28% increase in cell proliferation rate compared to those cultured in the medium without FGF-2 (FIG. 7b), but failed to exhibit a spheroid morphology (FIG. 7a).

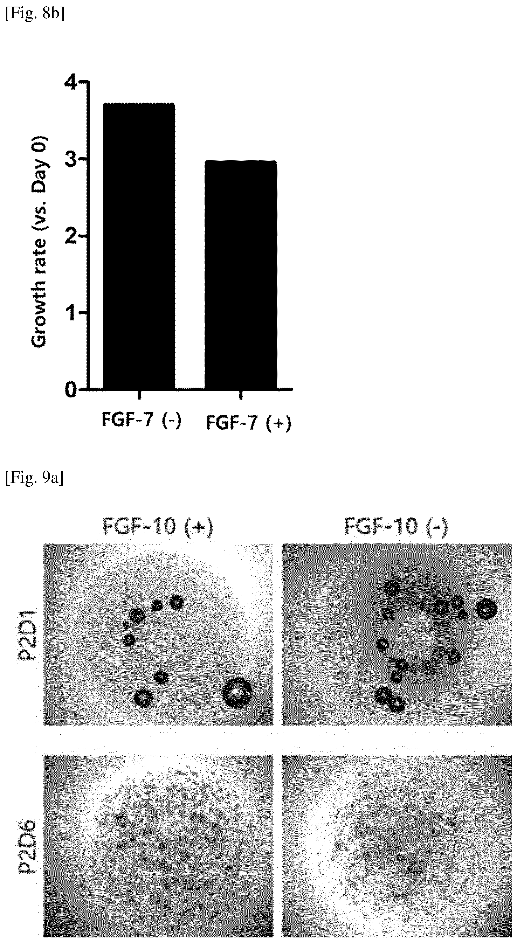

As shown in FIGS. 8a and 8b, salivary gland organoids cultured in the medium containing FGF-7 exhibited approximately a 20% decrease in cell proliferation rate compared to those cultured in the medium without FGF-7.

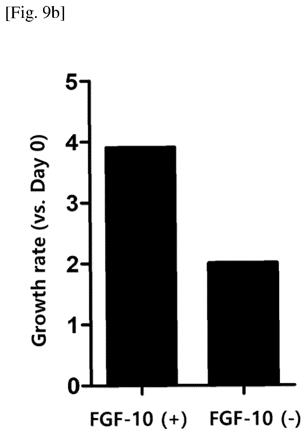

In addition, as shown in FIGS. 9a and 9b, salivary gland organoids cultured in the medium without FGF-10 exhibited approximately a 49% decrease in cell proliferation rate compared to those cultured in the medium containing FGF-10 (FIG. 9b), and also failed to exhibit a spheroid morphology (FIG. 9a).

These results suggest that when FGF-2 or FGF-7 is included, or when FSK or FGF-10 is excluded, the cell proliferation rate decreases and the cultured salivary gland organoids lose their spheroid morphology, indicating that it is important to exclude FGF-2 and FGF-7 and to include FSK and FGF-10 in the medium composition.

## Claims

1. A medium composition for preparing salivary gland organoids, comprising forskolin, FGF-10 and HRG-β1.

2. The medium composition according to claim 1, wherein the salivary gland organoids express one or more markers selected from the group consisting of EpCAM, CD29, E-cadherin and CD49f.

3. The medium composition according to claim 1, wherein the composition further comprises a basal medium.

4. The medium composition according to claim 3, wherein the basal medium is DMEM, MEM, BME, RPMI1640, F-10, F-12, α-MEM, GMEM, IMDM, DMEM/F12 or Advanced DMEM/F12.

5. The medium composition according to claim 1, wherein the composition further comprises one or more antibiotics selected from the group consisting of gentamicin, amphotericin and streptomycin.

6. The medium composition according to claim 1, wherein the composition does not comprise one or more selected from the group consisting of FGF-2 and FGF-7.

7. A method for preparing salivary gland organoids, comprising culturing salivary gland-derived cells isolated from a subject using the medium composition of claim 1.

8. Salivary gland organoids prepared by the method of claim 7.

9. A pharmaceutical composition for preventing or treating a salivary gland disease, comprising the salivary gland organoids of claim 8.

10. The pharmaceutical composition according to claim 9, wherein the salivary gland disease is one or more selected from the group consisting of sialadenitis, xerostomia, salivary gland tumor, salivary gland injury caused by irradiation or radioactive isotopes, and salivary gland dysfunction caused by aging.

11. The pharmaceutical composition according to claim 10, wherein the sialadenitis is one or more selected from the group consisting of tuberculous sialadenitis, infectious acute sialadenitis, infectious chronic sialadenitis, and sialadenitis caused by irradiation or radioactive isotopes.
